# EUROPEAN PATENT SPECIFICATION

(11) **EP 4 448 033 B1**
(45) Date of publication and mention of the grant of the patent: **25.11.2026**
(21) Application number: 21848422.8
(22) Date of filing: 28.12.2021
(51) Int. Cl.: A61L 29/00, A61L 29/04, A61L 29/08, A61M 39/02, A61M 39/04

(54) **COATED SUBCUTANEOUS ACCESS DEVICE AND ASSOCIATED METHODS**
BESCHICHTETE SUBKUTANE ZUGANGSVORRICHTUNG UND ZUGEHÖRIGE VERFAHREN
DISPOSITIF D'ACCÈS SOUS-CUTANÉ REVÊTU ET PROCÉDÉS ASSOCIÉS

(43) Date of publication of application: 23.10.2024
(73) Proprietor: Bard Peripheral Vascular, Inc., Franklin Lakes, NJ 07417 (US)
(72) Inventor: SHREVE, Samuel M., Scottsdale, AZ 85251 (US); EVANS, John G., South Jordan, UT 94095 (US)
(74) Representative: Hoffmann Eitle
(86) International application number: PCT/US2021/065323
(87) International publication number: WO 2023/129132

(56) References cited:
- WO-A1-2005/037338
- WO-A2-2014/172569
- US-A1- 2020 230 390

## Description

### BACKGROUND

US 2020/230390 A1 discloses a power injectable access port.

WO 2014/172569 A2 discloses antimicrobial catheters and medical devices. In some aspects, a low durometer aliphatic polyether polyurethane may be impregnated with a first antimicrobial agent (e.g., minocycline and rifampin) and coated with a second antimicrobial agent (e.g., chlorhexidine, gendine, or gardine).

### SUMMARY

Briefly summarized, embodiments disclosed herein are directed to a vascular access system including a port, catheter, cathlock, or combinations thereof, and including a coating disposed in a deactivated state on a surface thereof. The coating can include a lubricious and/or hydrophilic coating configured to reduce infection, reduce biofilm formation, and improve ease of insertion. Embodiments include active ingredients formed within the coating and/or can include custom dips to activate the lubricious coating and load one or more active ingredients into the coating prior to subcutaneous placement. The active ingredients can be configured to prevent infection, prevent thrombi formation, prevent biofilm formation, combinations thereof, or the like.

Placing a vascular access system, or "port," can generally include accessing the vasculature, placing a catheter, forming a tissue pocket, placing the port subcutaneously within the tissue pocket, and coupling the catheter to the port stem, before closing one or more insertion sites. The procedure can be susceptible to various complications including trauma, skin stretching or scar tissue formation during formation of the tissue pocket and/or placing of the catheter and port. Further, the placement procedure is susceptible to the introduction of pathogens leading to post placement infections and associated complications. Lastly, once the vascular access system has been placed, the presence of the foreign body placed subcutaneously can result in biofilm formation and or thrombosis formation.

Disclosed herein is a subcutaneous vascular access system including, an access port having a body defining a reservoir and a stem in fluid communication with the reservoir, and a coating disposed in a deactivated state on an outer surface of the access port and configured for, (i) submersing in a solution to transition the coating to an activated state, (ii) transitioning the coating to the activated state by absorbing an active ingredient from the solution, and (iii) eluting the active ingredient following subcutaneous implantation.

In some embodiments, the subcutaneous vascular access system further includes a catheter configured to engage the stem, the coating disposed on an outer surface of the catheter. In some embodiments, the subcutaneous vascular access system further includes a cathlock configured to secure the catheter to the stem, the coating disposed on an outer surface of the cathlock. In some embodiments, the coating is configured to be a lubricious coating in the activated state to facilitate insertion through an incision site. In some embodiments, the coating is configured for eluting the active ingredient over a period of 48 hours. In some embodiments, the coating includes a base layer bonded to the surface of the port, and a loose polymer matrix bonded to the base layer.

In some embodiments, the coating includes one or more of polyurethane, polyvinylpyrrolidone, hyaluronicacid, a UV Curable H-Coating, and a poly-N-vinylpyrrolidone H-coating. In some embodiments, the coating is between 100µm and 500µm thick. In some embodiments, the coating is configured for absorbing the active ingredient from the solution in a range of between two (2) mins and ten (10) mins. In some embodiments, the active ingredient includes one or more of an antibiotic agent, antifungal agent, anti-thrombotic agent, rifampin, gentamicin, minocycline, teicoplanin, glycopeptide teicoplanin, vancomycin, ceftazidime, and amphotericin B.

Also disclosed is a method of placing a subcutaneous vascular access assembly including, providing a vascular access assembly including an access port, a catheter, and a cathlock, the access port including a coating disposed on an outer surface thereof, the coating including a hydrophilic material, immersing a portion of the access port in a solution to transition the coating to an activated state, and subcutaneously implanting the vascular access assembly.

In some embodiments, the step of immersing further includes transitioning the coating to a lubricious coating in the activated state to facilitate insertion of the vascular access assembly through an incision site to place the vascular access assembly subcutaneously. In some embodiments, the solution includes an active ingredient, and the method further includes absorbing the active ingredient from the solution into the coating and eluting the active ingredient from the coating when placed subcutaneously.

In some embodiments, the active ingredient includes one or more of an antibiotic agent, antifungal agent, anti-thrombotic agent, rifampin, gentamicin, minocycline, teicoplanin, glycopeptide teicoplanin, vancomycin, ceftazidime, and amphotericin B. In some embodiments, the coating includes a base layer bonded to the surface of the vascular access assembly, and a loose polymer matrix bonded to the base layer. In some embodiments, the coating includes one or more of polyurethane, polyvinylpyrrolidone, hyaluronicacid, a UV Curable H-Coating, and a poly-N-vinylpyrrolidone H-coating. In some embodiments, the coating is between 100µm and 500µm thick. In some embodiments, the coating is submerged within the solution for between two (2) mins and ten (10) mins.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1A shows a perspective view of a vascular access system, in accordance with embodiments disclosed herein.
FIG. 1B shows an exploded view of the vascular access system of FIG. 1A, in accordance with embodiments disclosed herein.
FIGS. 2A-2C show an exemplary method of activating a coating disposed on a vascular access system, in accordance with embodiments disclosed herein.
FIG. 3 shows a schematic cross-section view of a coating disposed on a surface of a vascular access system, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

In the following description, the terms "or" and "and/or" as used herein are to be interpreted as inclusive or meaning any one or any combination. As an example, "A, B or C" or "A, B and/or C" mean "any of the following, A, B, C, A and B, A and C, B and C, A, B and C." An exception to this definition will occur only when a combination of elements, components, functions, steps or acts are in some way inherently mutually exclusive.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

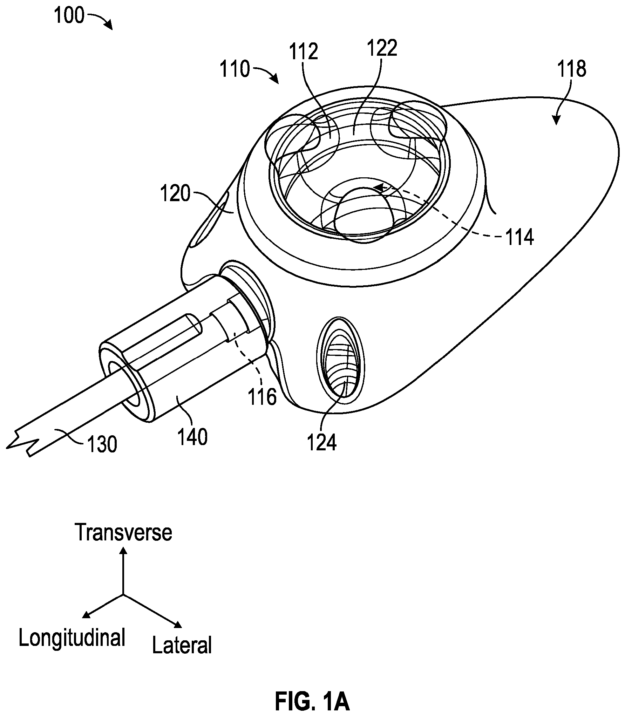

To assist in the description of embodiments described herein, as shown in FIGS. 1A-1B, a longitudinal axis extends substantially parallel to an axial length of the port stem. A lateral axis extends normal to the longitudinal axis, and a transverse axis extends normal to both the longitudinal and lateral axes. A horizontal plane is defined by the longitudinal and lateral axes. A vertical plane extends perpendicular to the horizontal axis.

Unless defined otherwise, all technical and scientific terms used herein have the same meaning as commonly understood by those of ordinary skill in the art.

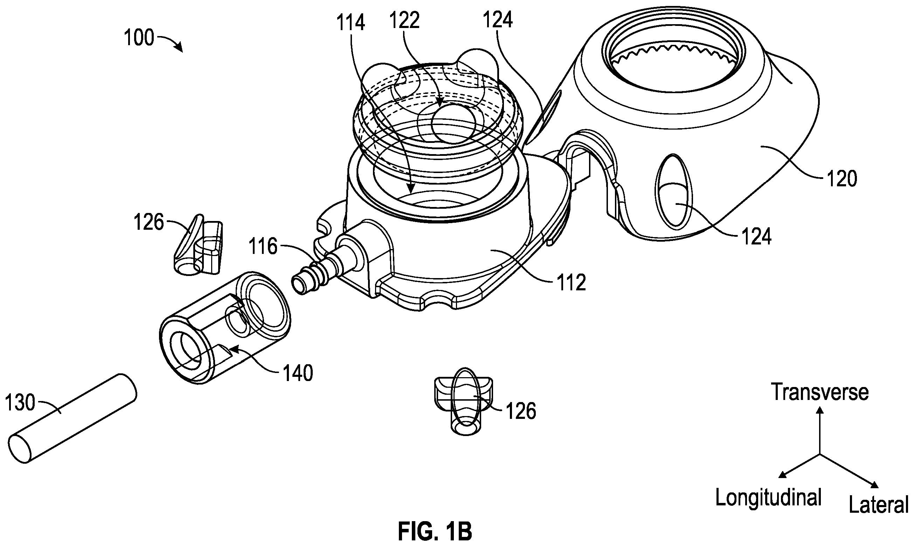

FIGS. 1A-1B show an exemplary subcutaneous vascular access system ("system") 100, generally including an access port ("port") 110, a catheter 130, and optionally a cathlock 140. FIG. 1A shows a perspective view of the system 100, FIG. 1B shows an exploded view of the system 100. The port 100 can generally include a body 112 that defines a reservoir 114, and includes a stem 116 extending therefrom along a longitudinal axis. The stem 116 can define a stem lumen that is in fluid communication with the reservoir 114. A needle penetrable septum 122 can be disposed over the reservoir 114 and can be secured in place by a cap 120. The septum 122 can be formed of a silicone rubber or similar suitable material. In use, when the port 110 is placed subcutaneously, septum 122 is disposed proximate a skin surface. A user can penetrate the skin using an access needle (not shown) and can penetrate a septum 122 to access the reservoir 112.

The cap 120 can engage the body 112 in a press-fit, interference fit, or snap fit engagement. In an embodiment, the cap 120 can be secured to the body 112 using adhesive, bonding, welding, or similar suitable means. In an embodiment, the cap 120 can be over-molded on to the body 112. In an embodiment, the cap 120 and the body 112 can be formed as a single monolithic piece formed of the same material.

In an embodiment, the body 112 can be formed of a substantially rigid or resilient material including, but not limited to, a plastic, polymer, thermoplastic, metal, alloy, composite, combinations thereof, or the like. In an embodiment, the cap 120 can be formed of a softer material relative to the body 112 including, but not limited to, a plastic, polymer, thermoplastic, elastomer, rubber, silicone rubber, metal, alloy, composite, combinations thereof, or the like. In an embodiment, the body 112 and the cap 120 can be formed of the same material and can display the same mechanical properties. In an embodiment, a catheter 130 can engage the stem 116 in one of a press-fit or interference fit engagement. Optionally a cathlock 140 can engage one or both of the catheter 130 and the stem 116 to further secure the catheter 140 to the stem 116.

In an embodiment, the port 110 can include a nose portion 118 extending from the port 110 along a longitudinal axis and disposed opposite the stem 116 across a central transverse axis of the reservoir 114. The nose portion 118 can define a substantially wedge shaped profile extending from the port 110 and can be configured to facilitate subcutaneous placement of the port 110. For example, as the port 110 is urged subcutaneously, the nose portion 118 can facilitate separation of subcutaneous tissues to form a tissue pocket. In an embodiment, one or both of the body 112 and the cap 120 can include a suture hole 124. The suture hole can include a plug 126 formed or silicone rubber, or similar suitable material and can be configured to prevent tissue ingrowth into the suture hole 124. As will be appreciated, the port 110 is an exemplary vascular access device and not intended to be limiting. As such, it is contemplated that various ports or access devices that include a rigid stem and configured to be coupled with a catheter or similar compliant tube are contemplated to fall within the scope of the present invention.

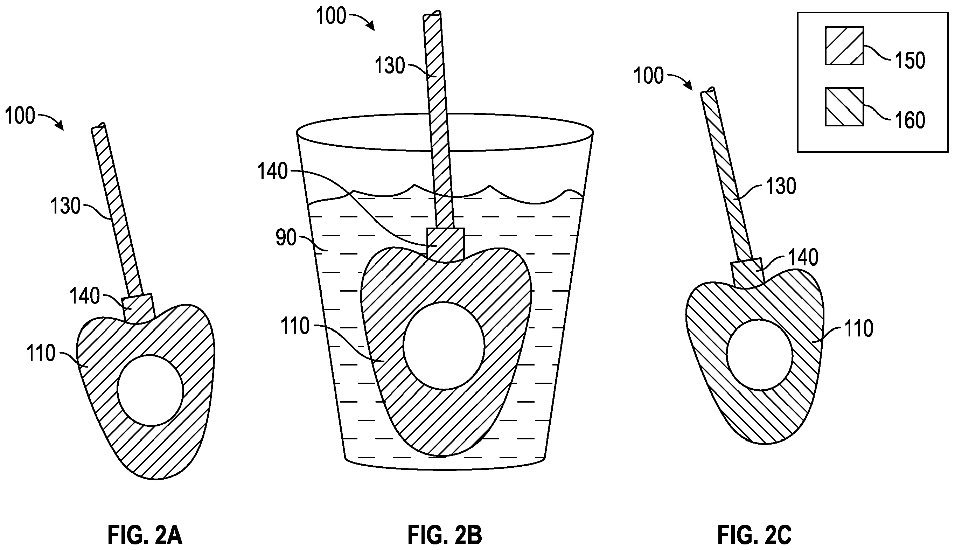

In an embodiment, as shown in FIG. 2A, an outer surface of the system 100, i.e. an outer surface of one or more of the catheter 130, cathlock 140, port 110, portions thereof, or combinations thereof, can include a coating 150 disposed thereon. In an embodiment, the coating 150 can be disposed over an entire outer surface of the system 100 including an outer surface of the catheter 130, cathlock 140, port 110 (i.e. body 112, cap 120, septum 122, etc.). In an embodiment, the coating 150 can be disposed on a portion of the system 100, for example over one or more portions of the system 100 that requires increased lubrication during placement (e.g. an outer surface of the port 110, cap 120, lower surface of the body 112, combinations thereof, or the like). In an embodiment, the coating 150 can be disposed on a portion of the system 100 that has an increased risk of causing infection or thrombosis formation, for example the suture holes 124, suture plugs 126, cathlock 140, or the like.

In an embodiment, the coating 150 can be a lubricious coating that is pre-wetted and configured to provide a reduced friction co-efficient to a surface of the system 100. Advantageously, the lubricious coating can facilitate inserting the system 100 through an insertion site in a skin surface of the patient, reducing stretching, trauma, and scar tissue formation. In an embodiment, the coating 150 can be a hydrophilic coating configured to transition between a deactivated state and an activated state. In an embodiment, a deactivated state can be a dehydrated state and an activated state can be a hydrated state. In an embodiment, a deactivated state can be defined as less than a threshold hydration level, and an activated state can be a defined as greater than a threshold hydration level. In an embodiment, a threshold hydration level can be between 1% and 99% hydration. In an embodiment, a threshold hydration level can be between 5% and 80% hydration. In an embodiment, a threshold hydration level can be substantially 50% hydration. However, it will be appreciated that greater or lesser hydration thresholds are contemplated.

In an embodiment, the coating 150 can transition from a deactivated state to an activated state by immersing the coating 150 in a solution 90. Exemplary solutions 90 can include water, saline, polar solutions, or similar biocompatible solutions. In an embodiment, a deactivated state can be defined as a dwell time of less than a threshold dwell time within the solution 90, and an activated state can be a defined as a dwell time as greater than a threshold dwell time. In an embodiment, a threshold dwell time can be between 1 secs and 10 mins. However, it will be appreciated that greater or lesser dwell time thresholds are contemplated. Once the (deactivated) hydrophilic coating 150 has been transitioned to an activated coating 160, the coating can provide a lubricious coating to provide a reduced friction co-efficient to a surface of the system 100. Advantageously, the activated hydrophilic coating 160 can facilitate inserting the system 100, through an insertion site in a skin surface of the patient reducing stretching, trauma, and scar tissue formation. Advantageously, the deactivated coating can be stored and transported in a dehydrated state extending the storage life, or "shelf-life," of the device.

In an embodiment, the coating 150 can include an active ingredient formed within the coating during manufacture. Exemplary active ingredients can include antibiotic agents, antifungal agents, anti-thrombotic agents, rifampin, gentamicin, minocycline, teicoplanin, glycopeptide teicoplanin, vancomycin, ceftazidime, and amphotericin B, or combinations thereof.

In an embodiment, a deactivated state can be defined as a concentration of an active ingredient of less than a threshold concertation, and an activated state can be a defined as a concentration of an active ingredient greater than a threshold concertation. In an embodiment, a threshold concentration can be equal to or greater than a zero concentration of an active ingredient.

In an embodiment, the coating 150 can be submersed in the solution 90 and can absorb one or more of water and active ingredients from the activation solution 90, to transition the coating from a deactivated state coating 150 to an activated state coating 160. The activated state coating 160 can be a lubricious to facilitate subcutaneous placement. Further, once placed subcutaneously, the activated state coating 160 can elute the active into the surrounding tissues. As such, the activated state coating 160 can act as a reservoir for the one or more active ingredients, providing extended elution times for the active ingredient once the system 100 is placed subcutaneously.

For example, conventional methods of placing a vascular access system 100 can include washing the tissue pocket with a solution including an active ingredient prior to placing the port 110. The active ingredient can be configured to prevent infection, biofilm formation, thrombosis formation, or the like. However, the efficacy of the solution decreases sharply, over time, from the point where the clinician finishes washing the tissue pocket. Advantageously, embodiments disclosed herein provide a coating 150 that can act as a reservoir for one or more active ingredients. The active ingredients can be eluted into the surrounding tissues over an extended period of time, extending the efficacy of the active ingredient. Exemplary elution times can be up to 48 hours after the placement of the system 100, however greater or lesser elution times are also contemplated.

In an embodiment, the system 100 including the coating 150 can be disposed within the solution 90 transitioning the coating 150 to an activated coating 160. During activation of the coating, one or more active ingredients dissolved within the solution 90 can be loaded into the coating. Advantageously, a clinician can select one or more active ingredients, different concentrations of the active ingredients, and/or the length of dwell time the system 100 remains within the solution 90. As such, the clinician can modify the active ingredients and elution rates for the system 100 depending on the specific procedure being performed or the specific needs of the patient. As will be appreciated, such a system allows for a single device to be manufactured, transported and stored and the modified at the point of use to be applied to various different procedures and patients. This can provide a more versatile system 100 and prevent manufacturing and storing large inventories of different vascular access systems, reducing associated costs. Similarly, when activating the coating 150 to an activated state 160, a clinician can modify the dwell time of the system 100 within the solution 90 to modify a degree of lubricity of the system 100 as required by the procedure or needs of the patient. Similarly, this can provide a more versatile system 100 reducing excess inventory and associated costs.

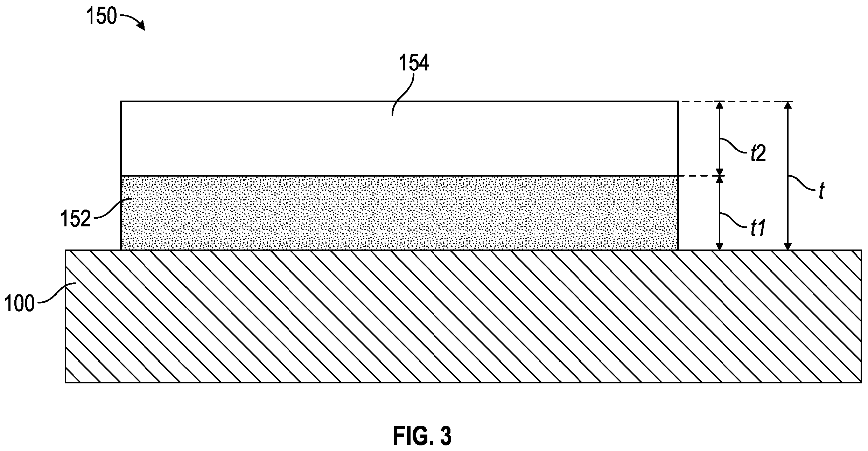

FIG. 3 shows a schematic cross-section view of the coating 150 disposed on a surface of the system 100. In an embodiment, the coating 150 can include one or more layers. In an embodiment, the coating 150 can include a base layer 152 that is bonded to the surface of the system 100. The coating 150 can further include a second layer 154 that is then bonded to the base layer 152. In an embodiment, the second layer 154 can include a loose polymer matrix. In an embodiment, the loose polymer matrix layer 154 can be a hydrophilic material configured to absorb water, saline solution, or polar solutions, and any active ingredients (e.g. antibiotics, etc.) dissolved therein. In an embodiment, the coating 150 can include one or more of polyurethane, polyvinylpyrrolidone, hyaluronicacid, a UV Curable H-Coating, and a poly-N-vinylpyrrolidone H-coating, or similar hydrophilic materials.

In an embodiment, the degree of lubricity (i.e. friction coefficient), or the rate of active ingredient elution can be varied based on the thickness of the coating (t), or the relative thickness of one or more layers of the coating 150, i.e. the thickness of the base layer 152 (*t1*) or the thickness of the polymer matrix 154 (*t2*)*,* or combinations thereof. Exemplary coating 150 thickness (t) can be between 100µm and 500µm.

FIGS. 2A-2C show an exemplary method of use for the system 100 including the coating 150, as described herein. As shown in FIG. 2A a vascular access system 100 can be provided including a port 110, catheter 130, cathlock 140, or combinations thereof, as described herein. A coating 150 can be provided on at least a portion of an outer surface of the system 100. In an embodiment, the coating 150 can be disposed over an entire surface of the system 100. In an embodiment, as shown in FIG. 2A, the coating 150 can be a lubricious coating 150 and/or can include an active ingredient formed within the coating 150.

In an embodiment, as shown in FIGS. 2B-2C, the coating 150 can be a hydrophilic coating 150 that can be transitioned to an activated coating 160 when placed within solution 90. In an embodiment, transitioning the coating 150 to an activated coating 160 can include transitioning a dehydrated coating 150 to a hydrated, or lubricious, coating 160. In an embodiment, transitioning the coating 150 to an activated coating 160 can include loading the coating 150 with an active ingredient to provide a drug eluting coating 160. In an embodiment, the coating 150 prior to activation can have no active ingredients disposed therein and can have one or more active ingredients loaded when placed within the solution 90. In an embodiment, the coating 150 can have one or more first active ingredient(s) disposed within the coating 150 prior to activation. During activation, one or more second active ingredient(s) can be loaded into the coating as described herein.

In an embodiment, a clinician can modify the degree of lubriciousness based on the length of dwell time within the solution 90. Exemplary dwell times can be between two (2) mins and ten (10) mins, however greater or lesser dwell times are also contemplated. In an embodiment, a clinician can modify the drug elution time based on the length of dwell time within the solution 90 including the active ingredient. Exemplary dwell times can be between two (2) mins and ten (10) mins, however greater or lesser dwell times are also contemplated.

In an embodiment, a clinician can modify the number or combination of active ingredients in the coating by modifying the number, combination, or relative concentrations of active ingredients within the solution 90. Advantageously, the clinician can modify the degree of lubriciousness or active ingredient combinations at the point of placement based on the specifics of the procedure or various patient factors, or the like. This provides a more versatile system 100 that can be modified to suit various patient factors, reducing device inventory and associated costs. Advantageously, embodiments described herein allow for a system 100 to be modified at the point of placement with various combinations of active ingredients or degrees of lubricity and provide increased efficacy through the synergistic effects of the active ingredient combination. Advantageously, the coating can provide extended drug elution times by providing a reservoir for active ingredients, relative to washing the tissue pocket and/or system 100 with active ingredients prior to placement. For example, the system 100 can provide elution times of up to 48 hours after placement or longer.

While some particular embodiments have been disclosed herein, and while the particular embodiments have been disclosed in some detail, it is not the intention for the particular embodiments to limit the scope of the concepts provided herein.

## Claims

1. A subcutaneous vascular access system (100), comprising:
an access port (110) having a body (112) defining a reservoir (114) and a stem (116) in fluid communication with the reservoir (114); and
a coating (150) disposed on an outer surface of the access port (110) in a deactivated state and configured for:
(i) submersing in a solution (90) to transition the coating (150) to an activated state;
(ii) transitioning the coating to the activated state by absorbing an active ingredient from the solution (90); and
(iii) eluting the active ingredient following subcutaneous implantation.

2. The subcutaneous vascular access system (100) according to claim 1, further including a catheter (130) configured to engage the stem (116), the coating (150) disposed on an outer surface of the catheter (130), optionally further including a cathlock (140) configured to secure the catheter (130) to the stem (116), the coating (150) disposed on an outer surface of the cathlock (140).

3. The subcutaneous vascular access system (100) according to any one of claims 1-2, wherein the coating (150) is configured to be a lubricious coating in the activated state to facilitate insertion through an incision site.

4. The subcutaneous vascular access system (100) according to any one of claims 1-3, wherein the coating (150) is configured for eluting the active ingredient over a period of 48 hours.

5. The subcutaneous vascular access system (100) according to any one of claims 1-4, wherein the coating (150) includes a base layer (152) bonded to the surface of the port (110), and a loose polymer matrix bonded to the base layer (152).

6. The subcutaneous vascular access system (100) according to any one of claims 1-5, wherein the coating (150) includes one or more of polyurethane, polyvinylpyrrolidone, hyaluronicacid, a UV Curable H-Coating, and a poly-N-vinylpyrrolidone H-coating.

7. The subcutaneous vascular access system (100) according to any one of claims 1-6, wherein the coating (150) is between 100µm and 500µm thick.

8. The subcutaneous vascular access system (100) according to any one of claims 1-7, wherein the coating (150) is configured for absorbing the active ingredient from the solution (90) in a range of between two minutes and ten minutess.

9. The subcutaneous vascular access system (100) according to any one of claims 1-8, wherein the active ingredient includes one or more of an antibiotic agent, antifungal agent, anti-thrombotic agent, rifampin, gentamicin, minocycline, teicoplanin, glycopeptide teicoplanin, vancomycin, ceftazidime, and amphotericin B.

10. A method of using a subcutaneous vascular access assembly, comprising:
providing a vascular access assembly comprising an access port (110), a catheter (130), and a cathlock (140), the access port (110) including a coating (150) disposed on an outer surface thereof in a deactivated state, the coating (150) including a hydrophilic material;
immersing a portion of the access port (110) in a solution (90) to transition the coating (150) to an activated state.

11. The method according to claim 10, wherein the step of immersing further includes transitioning the coating (150) to a lubricious coating in the activated state to facilitate insertion of the vascular access assembly through an incision site to place the vascular access assembly subcutaneously.

12. The method according to any one of claims 10-11, wherein the solution (90) includes an active ingredient, and further includes absorbing the active ingredient from the solution (90) into the coating (150) and eluting the active ingredient from the coating (150) when placed subcutaneously, optionally wherein the active ingredient includes one or more of an antibiotic agent, antifungal agent, anti-thrombotic agent, rifampin, gentamicin, minocycline, teicoplanin, glycopeptide teicoplanin, vancomycin, ceftazidime, and amphotericin B.

13. The method according to any one of claims 10-12, wherein the coating (150) includes a base layer (152) bonded to the surface of the vascular access assembly, and a loose polymer matrix bonded to the base layer (152); and/or wherein the coating (150) includes one or more of polyurethane, polyvinylpyrrolidone, hyaluronicacid, a UV Curable H-Coating, and a poly-N-vinylpyrrolidone H-coating.

14. The method according to any one of claims 10-13, wherein the coating (150) is between 100µm and 500µm thick.

15. The method according to any one of claims 10-14, wherein the coating (150) is submerged within the solution (90) for between two minutes and ten minutes.

## Patentansprüche

1. Subkutanes Gefäßzugangssystem (100), umfassend:
einen Zugangsport (110), der einen Körper (112), der einen Behälter (114) definiert, und einen Schaft (116) in Fluidverbindung mit dem Behälter (114) aufweist; und
eine Beschichtung (150), die in einem deaktivierten Zustand auf einer Außenoberfläche des Zugangsports (110) angeordnet und ausgebildet ist zum:
(i) Eintauchen in eine Lösung (90), um die Beschichtung (150) in einen aktivierten Zustand zu überführen;
(ii) Überführen der Beschichtung in den aktivierten Zustand durch Absorbieren eines Wirkstoffs aus der Lösung (90); und
(iii) Freisetzen des Wirkstoffs nach subkutaner Implantation.

2. Subkutanes Gefäßzugangssystem (100) nach Anspruch 1, weiter einschließend einen Katheter (130), der dazu ausgebildet ist, mit dem Schaft (116) in Eingriff zu kommen, wobei die Beschichtung (150) auf einer Außenoberfläche des Katheters (130) angeordnet ist, optional weiter einschließend ein Cathlock (140), das dazu ausgebildet ist, den Katheter (130) an dem Schaft (116) zu sichern, wobei die Beschichtung (150) auf einer Außenoberfläche des Cathlocks (140) angeordnet ist.

3. Subkutanes Gefäßzugangssystem (100) nach einem der Ansprüche 1-2, wobei die Beschichtung (150) dazu ausgebildet ist, in dem aktivierten Zustand eine gleitfähige Beschichtung zu sein, um das Einführen durch eine Inzisionsstelle hindurch zu erleichtern.

4. Subkutanes Gefäßzugangssystem (100) nach einem der Ansprüche 1-3, wobei die Beschichtung (150) dazu ausgebildet ist, den Wirkstoff über einen Zeitraum von 48 Stunden freizusetzen.

5. Subkutanes Gefäßzugangssystem (100) nach einem der Ansprüche 1-4, wobei die Beschichtung (150) eine an die Oberfläche des Ports (110) gebundene Grundschicht (152) und eine an die Grundschicht (152) gebundene lockere Polymermatrix einschließt.

6. Subkutanes Gefäßzugangssystem (100) nach einem der Ansprüche 1-5, wobei die Beschichtung (150) eines oder mehrere von Polyurethan, Polyvinylpyrrolidon, Hyaluronsäure, einer UV-härtbaren H-Beschichtung und einer Poly(N-vinylpyrrolidon)-H-Beschichtung einschließt.

7. Subkutanes Gefäßzugangssystem (100) nach einem der Ansprüche 1-6, wobei die Beschichtung (150) zwischen 100 µm und 500 µm dick ist.

8. Subkutanes Gefäßzugangssystem (100) nach einem der Ansprüche 1-7, wobei die Beschichtung (150) zum Absorbieren des Wirkstoffs aus der Lösung (90) innerhalb eines Zeitraums von zwei Minuten bis zehn Minuten ausgebildet ist.

9. Subkutanes Gefäßzugangssystem (100) nach einem der Ansprüche 1-8, wobei der Wirkstoff eines oder mehrere von einem antibiotischen Mittel, einem antimykotischen Mittel, einem antithrombotischen Mittel, Rifampicin, Gentamicin, Minocyclin, Teicoplanin, Glykopeptid-Teicoplanin, Vancomycin, Ceftazidim und Amphotericin B einschließt.

10. Verfahren zum Verwenden einer subkutanen Gefäßzugangsbaugruppe, umfassend:
Bereitstellen einer Gefäßzugangsbaugruppe, die einen Zugangsport (110), einen Katheter (130) und ein Cathlock (140) umfasst, wobei der Zugangsport (110) eine Beschichtung (150) einschließt, die in einem deaktivierten Zustand auf einer Außenoberfläche davon angeordnet ist, und wobei die Beschichtung (150) ein hydrophiles Material einschließt;
Eintauchen eines Abschnitts des Zugangsports (110) in eine Lösung (90), um die Beschichtung (150) in einen aktivierten Zustand zu überführen.

11. Verfahren nach Anspruch 10, wobei der Schritt des Eintauchens weiter Überführen der Beschichtung (150) in eine gleitfähige Beschichtung in dem aktivierten Zustand einschließt, um Einführen der Gefäßzugangsbaugruppe durch eine Inzisionsstelle hindurch zu erleichtern, um die Gefäßzugangsbaugruppe subkutan zu platzieren.

12. Verfahren nach einem der Ansprüche 10-11, wobei die Lösung (90) einen Wirkstoff einschließt, und weiter einschließend Absorbieren des Wirkstoffs aus der Lösung (90) in die Beschichtung (150) und Freisetzen des Wirkstoffs aus der Beschichtung (150), wenn sie subkutan platziert ist, wobei optional der Wirkstoff eines oder mehrere von einem antibiotischen Mittel, einem antimykotischen Mittel, einem antithrombotischen Mittel, Rifampicin, Gentamicin, Minocyclin, Teicoplanin, Glykopeptid-Teicoplanin, Vancomycin, Ceftazidim und Amphotericin B einschließt.

13. Verfahren nach einem der Ansprüche 10-12, wobei die Beschichtung (150) eine an die Oberfläche der Gefäßzugangsbaugruppe gebundene Grundschicht (152) und eine an die Grundschicht (152) gebundene lockere Polymermatrix einschließt; und/oder wobei die Beschichtung (150) eines oder mehrere von Polyurethan, Polyvinylpyrrolidon, Hyaluronsäure, einer UV-härtbaren H-Beschichtung und einer Poly-N-vinylpyrrolidon-H-Beschichtung einschließt.

14. Verfahren nach einem der Ansprüche 10-13, wobei die Beschichtung (150) zwischen 100 µm und 500 µm dick ist.

15. Verfahren nach einem der Ansprüche 10-14, wobei die Beschichtung (150) über einen Zeitraum von zwei Minuten bis zehn Minuten in die Lösung (90) eingetaucht wird.

## Revendications

1. Système d'accès vasculaire sous-cutané (100), comprenant :
un port (110) d'accès présentant un corps (112) définissant un réservoir (114) et une tige (116) en communication fluidique avec le réservoir (114) ; et
un revêtement (150) disposé sur une surface externe du port (110) d'accès dans un état désactivé et configuré pour :
(i) être immergé dans une solution (90) afin de faire passer le revêtement (150) à un état activé ;
(ii) faire passer le revêtement à l'état activé par absorption d'un principe actif à partir de la solution (90) ; et
(iii) éluer le principe actif après implantation sous-cutanée.

2. Système d'accès vasculaire sous-cutané (100) selon la revendication 1, incluant en outre un cathéter (130) configuré pour venir en prise avec la tige (116), le revêtement (150) étant disposé sur une surface externe du cathéter (130), incluant facultativement en outre un verrou de cathéter (140) configuré pour fixer le cathéter (130) à la tige (116), le revêtement (150) étant disposé sur une surface externe du verrou de cathéter (140).

3. Système d'accès vasculaire sous-cutané (100) selon l'une quelconque des revendications 1 à 2, dans lequel le revêtement (150) est configuré pour être un revêtement lubrifiant à l'état activé afin de faciliter l'insertion à travers un site d'incision.

4. Système d'accès vasculaire sous-cutané (100) selon l'une quelconque des revendications 1 à 3, dans lequel le revêtement (150) est configuré pour éluer le principe actif sur une période de 48 heures.

5. Système d'accès vasculaire sous-cutané (100) selon l'une quelconque des revendications 1 à 4, dans lequel le revêtement (150) inclut une couche de base (152) liée à la surface du port (110), et une matrice polymère lâche liée à la couche de base (152).

6. Système d'accès vasculaire sous-cutané (100) selon l'une quelconque des revendications 1 à 5, dans lequel le revêtement (150) inclut un ou plusieurs parmi le polyuréthane, la polyvinylpyrrolidone, l'acide hyaluronique, un revêtement H durcissable par UV et un revêtement H en poly-N-vinylpyrrolidone.

7. Système d'accès vasculaire sous-cutané (100) selon l'une quelconque des revendications 1 à 6, dans lequel le revêtement (150) est entre 100 µm et 500 µm d'épaisseur.

8. Système d'accès vasculaire sous-cutané (100) selon l'une quelconque des revendications 1 à 7, dans lequel le revêtement (150) est configuré pour absorber le principe actif à partir de la solution (90) dans une plage comprise entre deux minutes et dix minutes.

9. Système d'accès vasculaire sous-cutané (100) selon l'une quelconque des revendications 1 à 8, dans lequel le principe actif inclut un ou plusieurs parmi un agent antibiotique, un agent antifongique, un agent antithrombotique, la rifampicine, la gentamicine, la minocycline, la téicoplanine, le glycopeptide téicoplanine, la vancomycine, la ceftazidime et l'amphotéricine B.

10. Procédé d'utilisation d'un ensemble accès vasculaire sous-cutané, comprenant :
la fourniture d'un ensemble accès vasculaire comprenant un port (110) d'accès, un cathéter (130) et un verrou de cathéter (140), le port (110) d'accès incluant un revêtement (150) disposé sur une surface externe de celui-ci dans un état désactivé, le revêtement (150) incluant un matériau hydrophile ;
l'immersion d'une portion du port (110) d'accès dans une solution (90) pour faire passer le revêtement (150) à un état activé.

11. Procédé selon la revendication 10, dans lequel l'étape d'immersion inclut en outre la transition du revêtement (150) vers un revêtement lubrifiant à l'état activé afin de faciliter l'insertion de l'ensemble accès vasculaire à travers un site d'incision pour faire passer l'ensemble accès vasculaire par voie sous-cutanée.

12. Procédé selon l'une quelconque des revendications 10 à 11, dans lequel la solution (90) inclut un principe actif, et inclut en outre l'absorption du principe actif à partir de la solution (90) dans le revêtement (150) et l'élution du principe actif à partir du revêtement (150) lorsqu'il est placé par voie sous-cutanée, facultativement dans lequel le principe actif inclut un ou plusieurs parmi un agent antibiotique, un agent antifongique, un agent antithrombotique, la rifampicine, la gentamicine, la minocycline, la téicoplanine, le glycopeptide téicoplanine, la vancomycine, la ceftazidime, et l'amphotéricine B.

13. Procédé selon l'une quelconque des revendications 10 à 12, dans lequel le revêtement (150) inclut une couche de base (152) liée à la surface de l'ensemble accès vasculaire, et une matrice polymère lâche liée à la couche de base (152) ; et/ou dans lequel le revêtement (150) inclut un ou plusieurs parmi le polyuréthane, la polyvinylpyrrolidone, l'acide hyaluronique, un revêtement H durcissable par UV et un revêtement H en poly-N-vinylpyrrolidone.

14. Procédé selon l'une quelconque des revendications 10 à 13, dans lequel le revêtement (150) est entre 100 µm et 500 µm d'épaisseur.

15. Procédé selon l'une quelconque des revendications 10 à 14, dans lequel le revêtement (150) est immergé dans la solution (90) pendant entre deux minutes et dix minutes.
